# FASCICULE DE BREVET EUROPEEN

(11) **EP 0 471 804 B1**
(45) Date de publication et mention de la délivrance du brevet: **17.07.2002**
(21) Numéro de dépôt: 91904077.4
(22) Date de dépôt: 06.02.1991
(51) Int. Cl.: C12N 15/11, C12N 15/12, G01N 33/576

(54) **NOUVELLES COMPOSITIONS DE CYCLINE A HUMAINE ET LEUR PROCEDE DE PRODUCTION, SEQUENCE NUCLEOTIDIQUE CORRESPONDANTE, PROCEDE ET AGENTS DE DETECTION OU DE DIAGNOSTIC DE LA PROLIFERATION CELLULAIRE ET PROCEDE ET AGENTS D'INHIBITION DE LA PROLIFERATION CELLULAIRE**
MENSCHLICHE CYCLIN-A-ZUSAMMENSETZUNGEN, VERFAHREN ZU IHRER HERSTELLUNG, NUKLEOTIDSEQUENZ, VERFAHREN UND WIRKSTOFFE ZUR BESTIMMUNG ODER DIAGNOSE VON ZELLVERMEHRUNG, VERFAHREN UND WIRKSTOFFE ZUR HEMMUNG DER ZELLVERMEHRUNG
NOVEL HUMAN CYCLIN A COMPOSITIONS AND THEIR PRODUCTION METHOD, A CORRESPONDING NUCLEOTIDE SEQUENCE, A METHOD AND AGENTS FOR DETECTING OR DIAGNOSING CELL MULTIPLICATION, AND A METHOD AND AGENTS FOR INHIBITING CELL MULTIPLICATION

(30) Priorité: 12.02.1990 FR 9001596
(43) Date de publication de la demande: 26.02.1992
(73) Titulaire: INSTITUT NATIONAL DE LA SANTE ET DE LA RECHERCHE MEDICALE (INSERM), 75654 Paris Cédex 13 (FR)
(72) Inventeur: BRECHOT, Christian, F-75730 Paris Cédex 15 (FR); WANG, Jian, F-75730 Paris Cédex 15 (FR); CHENIVESSE, Xavier, F-75730 Paris Cédex 15 (FR); HENGLEIN, Berthold, F-75730 Paris Cédex 15 (FR); ZINDY, Frédérique, F-75730 Paris Cédex 15 (FR)
(74) Mandataire: Bernasconi, Jean
(86) Numéro de dépôt international: FR9100089
(87) Numéro de publication internationale: WO9112324

(56) Documents cités:
- Nature, vol. 343, 8 février 1990, (Londres,, GB), J. Wang et al.: "Hepatitis B virus integration in a cyclin A gene in a hepatocellular carcinoma", pages 555-557
- Idem.
- The American Journal of Pathology, vol. 134, no. 4, avril 1989, American Association of Pathologists, R.L. Garcia et al.: "Analysis of proliferative grade using anti-PCNA/ Cyclin monoclonal antibodies in fixed, embedded tissues", pages 733-740
- Science, vol. 240, no. 4858, 10 juin 1988, (Washington, DC, US), D. Jaskulski et al.: "Inhibition of cellular proliferation by antisense oligodeoxynucleotides to PCNA cyclin", pages 1544-1546
- Leukemia Research, vol. 8, no. 2, 1984, Pergamon Press Ltd, (GB), J.E. Celis et al.: "Cyclin: a nuclear protein whose level correlates directly with the proliferative state of normal as well as transformed cells", pages 143-157
- Cell, vol. 47, 26 décembre 1986, Cell Press. (Cambridge, Mass., US), K.I. Swenson et al.: "The clam embryo protein cyclin A induces entry into M phase and the resumption of meiosis in Xenopus Oocytes", pages 861-870
- Experimental Cell Research, vol. 163, no. 2, avril 1986, Academic Press, Inc., (SE), M.R. Sadaie et al.: "Immunochemical and biochemical analysis of the proliferating cell nuclear antigen (PCNA) in HeLa cells", pages 423-433
- Nature, vol. 346, 23 aout 1990, (Londres, GB), J. Pines et al.: "Human cyclin A is adenovirus E1A-associated protein p60 and behaves differently from cyclin B", pages 760-763
- PATERLINI ET AL. CANCER RESEARCH vol. 53, 15 Janvier 1993, pages 235 - 238
- ZINDY ET AL. BIOCHEMICAL AND BIOPHYSICAL RESEARCH COMMUNICATIONS vol. 182, no. 3, 14 Février 1992, pages 1144 - 1154

## Description

La présente invention a trait à l'utilisation de compositions contenant, à une concentration élevée et/ou à un degré de pureté élevé, une cycline A humaine, lesdites compositions pouvant être utiles notamment pour préparer des agents de détection de la cycline, tels que notamment des antisérums ou des anticorps, monoclonaux ou non.

L'invention a égalenent trait à un ADNc anti-sens complémentaire de la séquence nucleotidique codant pour la cycline A humaine ainsi qu'à des vecteurs d'expression incorporant cet ADNc.

L'invention concerne également un procédé et des agents de détection ou de diagnostic de la prolifération cellulaire.

Enfin, l'invention concerne un procédé et des agents d'inhibition de la prolifération cellulaire.

On sait que l'infection chronique par le virus de l'hépatite B (HBV) est un facteur de risque du développement du carcinome hépatocellulaire (22-24) et il a été établi que l'ADN proviral du virus HBV s'intègre fréquemment dans le génome de cellules humaines tumorales du cancer primaire du foie (1-4). Toutefois, le rôle joué par cette intégration dans la carcinogénése du foie n'est pas encore éclairci et les preuves d'un rôle direct du virus dans la transformation cellulaire sont rares. Plus particulièrement, les effets de l'intégration, de l'ADN viral dans le génome cellulaire sont controversés. Chez la marmotte d'Amérique, le virus de l'hépatite de la marmotte (WHV) s'est avéré agir souvent par dérégulation du MYC après l'insertion virale (25). Chez l'homme, l'intégration de HBV induit fréquemment, d'une manière non spécifique semble-t-il, des délétions et translocations (3, 26-28). Il a cependant été suggéré que l'intégration sur le chromosome 11 (3) ainsi que le réarrangement sur un locus du chromosome 4q (29, 30) se produisent de façon non aléatoire. L'intégration de HBV au voisinage d'un gène cellulaire a été démontrée uniquement dans une tumeur dans laquelle l'ADN viral a interrompu la région codant pour le récepteur béta de l'acide rétinoïque (31, 32).

J. Wang et al., Nature 343 : 555-557, 8 février 1990, ont décrit l'existence d'un site individualisè d'intégration de l'ADN du virus HBV dans le génome humain (chromosome 4q27) et plus précisément dans un gène codant pour une cycline A humaine dont l'existence a été découverte à la même occasion; cette intégration est présente dans les cellules hépatiques à un état précoce du développement tumoral sans réarrangement chromosomique notable, ne présentant aucune des caractéristiques histologiques de l'hépatite chronique ou de la cirrhose, dans une prolifération clonale d'une cellule contenant une seule intégration de HBV, absente dans le tissu hépatique adjacent, de sorte qu'il est plus que vraisemblable, par le dérèglement de la fonction de la cycline A qui en resulte, que cette insertion est directement impliquée dans la carcinogénése hépatique.

Ce document divulgue la séquence nucléotidique codant pour cette cycline A et rappelle que les cyclines interviennent lors de la phase G2/M du cycle cellulaire.

Les inventeurs ont découvert que l'expression de la cycline A (ARNm et protéine) apparaissait au moment de la phase S et de la phase G2/M du cycle cellulaire et était donc associée à la division cellulaire . Ainsi, ils ont trouvé, dans le foie de rat en régénération après hépatectomie partielle conduisant à une synthèse d'ADN cellulaire dans une forte proportion d'hépatocytes, une expression maximum de cycline A (ARNm et protéine) au moment de la phase de synthèse S de l'ADN cellulaire.

De même, les inventeurs ont constaté que l'expression de la cycline A était maximum dans les leucémies aiguës myéloblastiques hyperleucocytaires à temps de doublement rapide et très faible dans le cancer primitif du foie pour lequel le temps de doublement de la tumeur est lent.

Les inventeurs ont également découvert qu'il était possible de bloquer la synthèse de cycline A dans des hépatocytes a l'aide d'un ADN cycline A humaine "anti-sens".

Conformément à l'invention, on a cloné l'ADN correspondant au locus d'insertion et on a construit des vecteurs d'expression permettant de produire en grande quantité la cycline A codée. L'invention permet la préparation de cycline A humaine par expression génétique ainsi que l'obtention de compositions concentrées en cycline A humaine à un degré de pureté élevé.

La séquence nucléotidique isolée codant pour la cycline A humaine repérée No. : 1 sur la liste des séquences annexée présente une erreur de quelques nucléotides dans la partie N-terminale par rapport à la séquence divulguée dans J. Wang et al. (supra). Des vecteurs contenant et/ou exprimant la séquence codant pour la cycline A peuvent être préparés.

Cette séquence peut être associée à d'autres ensembles usuels tels que promoteurs, signaux d'initiation ou d'arrêt ou introns ou autres séquences non codantes à l'extrémité 3' et/ou 5' Elle inclut également les variantes obtenues notamment par substitution de codons ou de nucléotides conservant la signification du code, ou par substitutions, insertions ou délétions et codant pour un polypeptide équivalent et notamment conservant l'antigénicité du polypeptide. Elle inclut également tout fragment permettant l'expression d'un polypeptide conservant cette antigénicité. Enfin, la séquence s'étend également au gène de la cycline A humaine, incorporant ou non certains ou tous les éléments d'un opéron, et dépourvue de séquences flanquantes naturelles, et à tout fragment permettant l'expression d'un polypeptide conservant une antigénicité de la cycline A.

L'invention a pour objet un procédé de diagnostic in vitro de la prolifération cellulaire, dans lequel, à partir d'un échantillon de cellules ou de tissus, on détermine le taux de cycline A humaine, notamment à l'aide d'anticorps polyclonaux, par exemple des antisérums animaux induits par immunisation par la cycline A humaine ou des anticorps polyclonaux obtenus par expression de la protéine cycline A humaine dans des systèmes bactériens, ou des anticorps monoclonaux anticycline A humaine.

L'invention a donc aussi pour objet de tels anticorps.

L'invention a encore pour objet un procédé de traitement de la prolifération cellulaire, dans lequel on inhibe cette prolifération à l'aide d'ADNc cycline A humaine "anti-sens". Cette notion d'ADNc recouvre aussi les fragments pertinents de la séquence d'ADNc.

Pour assurer son introduction dans la cellule à traiter, cet ADNc peut être, notamment, soit inclus dans un vecteur rétroviral (par exemple selon les techniques décrites par J.A. Wolff et al., Proc. Natl. Acad. Sci.84 : 3344, 1987 ou par D.G. Miller et al., Mol. Cell. Biol. 10 : 4239, 1990), soit porté par une protéine désialilée capable de se fixer sur un récepteur porté par la cellule cible en vue de l'entrée de l'ADNc dans celle-ci, l'ADNc et la protéine étant notamment liés par une polylysine, (par exemple selon les techniques décrites par G.Y. Wu & G.H. Wu, J. Biol. Chem. 263 : 14621, 1988 et par G.H. Wu et al., J. Biol. Chem. 264: 16985, 1989), soit inclus dans un liposome.

L'invention a donc aussi pour objet l'ADNc cycline A anti-sens capable d'inhiber la prolifération cellulaire, les plasmides incluant cet ADNc et les vecteurs retroviraux l'incorporant.

L'invention a aussi pour objet le compose constitué par l'ADNc anti-sens et la protéine désialilée, ainsi que les liposomes incluant l'ADNc anti-sens.

D'autres avantages et caractéristiques de l'invention apparaîtront à la lecture de la description suivante, faite à titre d'exemple non limitatif.

### I Mise en évidence de l'insertion

J. Wang et al. (supra) ont analysé un carcinome hépatocellulaire primaire (HCC) précoce bien différencié développé sous forme d'un petit nodule isolé sans cirrhose d'accompagnement chez une femme présentant des anticorps contre l'antigène de surface de l'hépatite B. Ils ont extrait, selon la procédure classique, l'ADN cellulaire d'une part du tissu tumoral et d'autre part du tissu adjacent non tumoral. Les Southern blots de ces ADN ont été sondés par l'ADN de HBV. Alors que l'ADN de HBV, dans le tissu non tumoral, n'était présent que sous forme de molécules libres, l'ADN tumoral a formé une bande unique correspondant à l'intégration d'une seule copie de génome HBV par cellule.

Wang et al. ont construit une librairie phagique de l'ADN tumoral qui a permis d'isoler plusieurs clones chevauchants identifiés par une sonde spécifique HBV. La librairie a été construite par digestion partielle de l'ADN tumoral par Hind III et ligation dans les branches Hind III du phage L 47. Une population de clones de phages chevauchants a été isolée en utilisant HBV cloné comme sonde. Le sous-clonage a été effectué à l'aide de vecteurs Bluescript (Stratagène). L'allèle naturel a été cloné à partir d'une librairie EMBL 3 faite d'ADN placentaire, partiellement digéré par Sau 3A I. Le séquençage a été effectué par le procédé décrit dans la référence (39). L'ADNc a été isolé à partir d'une librairie construite à partir d'ARNm de foie humain adulte amorcé par oligo(dT) (32).

J. Wang et al. donnent la carte de restriction, qui en résulte, du provirus HBV et des séquences cellulaires flanquantes dans la tumeur et montrent les sous-clones A, X, Y, B, dépourvus de séquences répétitives, qui ont eté dérivés des clones phagiques. Le séquençage des fragments X et Y de la jonction provirus-hôte, avec l'analyse de restriction détaillée de l'insert proviral, ont confirmé l'obtention d'un génome viral sans réarrangements majeurs. Les jonctions virus-cellules sont toutes deux situées dans les régions d'extrémité de HBV (nucléotides 1840 et 1617 dans le noyau viral et les cadres de lecture ouverte X, respectivement (1). L'hybridation de Southern blots constitués à partir d'ADN tumoral par les sondes A et B a confirmé la configuration génomique. Avec la sonde B, c'est-à-dire un fragment EcoR I/Bgl IT de 1,1 kb, les auteurs ont balayé la librairie phagique du génome humain normal et isolé plusieurs clones chevauchants.

L'intégration virale dans la tumeur n'a pas entraîné de réarrangements majeurs dans son voisinage.

L'hybridation du clone B par Northern blots d'ARN polyadénylé et non polyadénylé de foie de nouveau-né normal a permis d'identifier deux molécules polyadénylées d'ARN transcrit de 1,8 kb et 2,7 kb. Le criblage consécutif d'une librairie phagique d'ADNc de foie humain normal a permis l'isolation de 5 clones d'ADNc qui, d'après leurs cartes de restriction, représentent la même molécule d'ARN. Les auteurs donnent la séquence d'ADNc de l'insert cloné le plus large.

Une recherche comparative effectuée sur ordinateur par les auteurs a montré que la protéine déduite de cet insert contient une région de 150 acides aminés comme boîte cycline (9) qui constitue le domaine majeur de conservation de toutes les cyclines connues (4, 5, 7, 9, 12). La protéine présente une grande ressemblance avec les cyclines A de S. solidissima (4) et D. melanogaster (5). Du fait qu'elle ne possède que 54 positions d'acides aminés, sur les 150 communes aux cases cycline des cyclines B de S. solidissima (1) et de l'homme (12), la protéine déduite est une cycline A humaine. L'extrémité N-terminale de la protéine ne montre pas d'homologie sensible à d'autres protéines connues, y compris d'autres cyclines, ce qui est une caractéristique commune à toutes les cyclines connues.

Le fragment A s'est avéré réagir par hybridation avec l'ADNc. Les auteurs ont donc partiellement séquencé les fragments A et B et trouvé des séquences à partir des extrémités 5' et 3' de l'ADNc respectivement. L'orientation qui s'en déduit du gène de la cycline A est de gauche a droite. L'étude de la séquence montre alors que la cycline A possède plusieurs exons et, du fait que les séquences cellulaires des fragments de jonction virus-hôte X,Y ne sont pas retrouvées dans l'ADNc, il en resulte que l'intégration de l'ADN du virus HBV s'effectue dans un intron du gène.

L'insertion virale dans le locus ainsi découvert dérègle le fonctionnement du gène de la cycline A, ce qui paraît avoir des conséquences décisives sur la régulation de la croissance cellulaire. Pour plus de détails sur les cyclines A et B, et leur importance dans la mitose, voir 5-21, 33-36.

### II Production de la cycline A humaine par expression genetique.

La molecule d'ADNc clone, représentée sur la liste, est insérée dans un vecteur d'expression bactérien ou de préférence eucaryote, par exemple par clonage de l'ADNc dans le vecteur de clonage Bluescript, insertion dans le vecteur plasmidique d'expression pET-3b, transfection d'un hôte, par exemple bactérien, les bouts de la séquence étant déterminés par les enzymes de restriction Eco RI et Sma I. Pour une description détaillée de cette technique, voir : Gène - 1987 , 56 : 125-135.

Une préparation de cycline A peut être obtenue à partir de la culture bactérienne par purification sur gel

### III Etude de l'expression du gène cycline A dans des hépatocytes et dans le foie en régénération

L'ADNc est utilisé comme sonde pour étudier l'expression de la cycline A sur le modèle des hépatocytes en culture et du foie de rat en régénération.

Des hépatocytes normaux de rat peuvent être maintenus en culture primaire pendant une période d'environ dix jours en gardant une partie des fonctions différenciées de l'hépatocyte. Il n'y a cependant pas prolifération des cellules. Il est possible de stimuler la synthèse d'ADN en ajoutant au milieu du facteur de croissance épidermique (épidermal growth factor EGF), du pyruvate et de l'insuline. On obtient une synthèse d'ADN cellulaire (mesurée par l'incorporation de thymidine tritiée) qui est maximale au troisième jour de culture. Il est apparu en Northern blot et en Western blot, que le pic d'accumulation d'ARN et de protéine cycline A se situe à la phase de synthèse de l'ADN (troisième jour de la culture après stimulation) contrairement à la cycline B1 qui est détectée dès le premier jour de mise en culture des hépatocytes jusqu'au cinquième jour. De plus, la cycline A n'est pas détectée dans des hépatocytes dont on n'a pas stimulé la synthèse d'ADN (contrairement à la cycline B1).

Afin de vérifier que ces observations in vitro reflétaient réellement la situation in vivo, on a utilisé le modèle du foie de rat en régénération. Après une hépatectomie des deux tiers, on assiste à une première mitose synchrone qui intéresse environ 40% des hépatocytes. Une synthèse d'ADN cellulaire entre la vingtième et la vingt-quatrième heure est suivie, environ six heures après, par la première mitose. Le point important est le déclenchement plus tardif de la prolifération des cellules non hépatocytaires du foie qui débutera environ vingt-quatre heures après celle des hépatocytes.

Il est apparu que :
- La cycline A n'est pas détectée dans un foie normal.
- La cycline A est détectée (ARN et protéine) au moment de la phase de synthèse de l'ADN cellulaire. L'accumulation de cycline A est suivie par une légère décroissance du taux de la cycline A, puis une réascension au moment de la mitose.
- Là encore, la cycline B1 a un profil tout à fait différent puisqu'elle est détectée dans un foie normal et que son taux n'augmente pas au moment de la phase S.

### IV Détection de cycline A sur des coupes de tissu obtenu par biopsie.

A partir de coupes congelées, on effectue une fixation au PFA (paraformaldéhyde) - PBS 4 % pendant 20 mn à 4°C ou au méthanol pendant 10 mn à -20°C, on lave au PBS, puis deux fois au Tris-HCl (pH 7,6).

A partir de coupes en paraffine, on déparaffine au xylène, on hydrate les coupes (à partir de l'alcool 100 % jusqu'à l'eau distillée), puis on lave à deux reprises au Tris-HCl (pH 7,6).

Le protocole devient ensuite :
- passage rapide en BSA (sérum albumine bovine) 1% ou Tris-HCl ;
- incubation avec de l'avidine, 15 mn ;
- lavage au Tris-HCl + passage en BSA ;
- incubation avec de la biotine, 15 mn ;
- 2 lavages au Tris-HCl + passage en BSA ;
- incubation avec du sérum normal, 20 mn ;
- élimination de l'excès de sérum ;
- incubation avec un anticorps anticycline A humaine une nuit à 4°C ;
- 2 lavages au Tris-HCl + passage en BSA ;
- incubation avec un anticorps de révélation biotinilé, pendant 30 mn;
- 2 lavages au Tris-HCl + passage en BSA ;
- incubation avec le complexe avidine-biotine, 30 mn;
- 2 lavages au Tris-HCl ;
- révélation par la diamino-benzidine (DAB), 5 mn au noir;
- lavages à l'eau ;
- coloration à l'hématoxiline ;
- déshydratation dans les alcools (jusqu'à 100 %) ;
- passages dans du xylène,
- lecture.

La lecture permet une certaine quantification du taux de cycline A présente dans le tissu par comptage du pourcentage des noyaux cellulaires qui ont été marqués. Dans le foie normal, le pourcentage est inférieur à 1 %. On a constaté des taux allant jusqu'à 80 % dans des tissus en prolifération.

### V Procédé de détection ou de diagnostic de la prolifération cellulaire.

Appliqué aux cellules circulantes, on peut recourir à toutes les méthodes bien connues de détection par anticorps, notamment ELISA et en particulier ELISA sandwich, appliquées à des prélèvements.

Appliqué aux tissus, on détecte la cycline A directement dans les tissus comme cela se fait de façon usuelle avec les outils de diagnostic de la prolifération connus.

La préparation d'anticorps anticycline A peut se faire chez le lapin par injection de la cycline A obtenue par purification sur gel en vue d'obtenir un anti-sérum de lapin.

La détection avec quantification de la cycline A humaine dans les tissus peut être ensuite réalisée par Western blot ou immunohistochimie.

Cette détection peut utiliser le comptage des noyaux cellulaires marqués et la comparaison du taux compté au taux d'un même tissu normal.

### VI Inhibition de la prolifération cellulaire.

In vitro, la microinjection, dans des hépatocytes, en culture primaire stimulés par l'insuline, le pyruvate et l'EGF, de plasmides contenant l'ADN complémentaire de la cycline A en situation anti-sens sous le contrôle d'éléments régulateurs du virus SV40, bloque la synthèse de l'ADN cellulaire.

Pour assurer in vivo l'inhibition de la prolifération cellulaire, notamment d'origine tumorale, on recourt à des constructions pour amener l'ADN anti-sens à l'intérieur de la cellule à traiter.

On peut d'abord recourir à un vecteur rétroviràl (J.A. Wolff et al. et D.G. Miller et al. déjà cités) incluant l'ADNc anti-sens et capable d'infecter la cellule cible et donc d'insérer l'ADNc anti-sens dans le génome de celle-ci. Pour ce faire, l'ADNc cycline A humaine est inséré en position anti-sens dans un plasmide, notamment le plasmide du commerce PKC4, avec un promoteur approprié, par exemple le promoteur du virus SV40. On sélectionne les plasmides intégrant bien en position anti-sens l'ADN à l'aide d'une carte de restriction avant d'introduire l'ADNc et son promoteur dans le rétrovirus.

On peut aussi utiliser une protéine capable de se fixer sur un récepteur de la cellule cible. On fixe l'ADNc anti-sens à une protéine désialilée (G.Y. Wu & C.H. Wu, et C.H. Wu et al. déjà cités) par l'intermédiaire d'une polylysine. Après fixation sur la cellule cible, l'ADNc est introduit dans le cytoplasme de la cellule dans lequel il est transcrit en ARN anti-sens qui bloquera la synthèse de cycline A par hybridation sur l'ARN cellulaire.

On peut encore recourir à des liposomes incluant l'ADNc anti-sens.

### Références

- 1: TIOLLAIS, P, POURCEL, C.& DEJEAN, A. Nature 317,489-495 (1985).
- 2: BRECHOT, C., POURCEL, C, LOUISE, A., RAIN, B.& TIOLLAIS, P Nature 286, 533-535 (1980).
- 3: NAGAYA, T. et al. Genes and Development l, 773-782(1987).
- 4 .: YAGINUMA, K. et al. J.Virol. 61, 1808-1813(1987).
- 5 .: SWENSON, K.I., FARRELL, K.M. & RUDERMAN, J.V. Cell 47, 861-870(1986).
- 6 .: LEHNER, C.F. & O'FARRELL, P. Cell 56, 957-968(1989).
- 7 .: STANDART, N., MINSHULL, J., PINES,J. & HUNT, T. Dev. Biol. 124, 248-258 (1987).
- 8 .: MINSHULL, J., BLOW, J.J. & HUNT T. Cell. 56, 947-956(1989).
- 9 .: MEIJER, L. et al. EMBO J. 8, 2275-2282(1989).
- 10 .: PINES. J. & HUNT, T. EMBO J. 6,2987-2995 (1987).
- 11 .: MURRAY, A.M. & KIRSCHNER, M.W. Natue 339, 275-286 (1989).
- 12 .: PINES, J. & HUNTER, T. Cell 58, 833-846 (1989).
- 13: SOLOMON, M., BOOHER, R., KIRSCHNER, M. & BEACH, D. Ceil 54,738-739 (1988)
- 14 .: GOEBL, M. & BYERS. B. Ceil 54, 739-740 (1988).
- 15 .: WESTENDORF, J.M., SWENSON,K.I. & RUDERMAN, J.V. J.Ceil. Biol. 108, 1431-1444 (1989).
- 16: DRAETTA, G. et al. Cell 56.29-838 (1989).
- 17: BRIZUELA, L., DRAETTA, G & BEACH, D. Proc. Natl. Acad. Sci. U.S.A. 86, 4362-4366 (1989).
- 18 .: DRAETTA, G. & BEACH, D. Cell 54, 17-26 (1989).
- 19 .: LABBE. J.C. et al. EMBO J.8, 3053-3058 (1989).
- 20 .: MORIA, A. O., DRAETTA, G., BEACH, D., WANG, J.Y.J. Cell 58, 193-203 (1989).
- 21 .: GIORDANO, A. et al. Cell 53, 981-990 (1989).
- 22 .: KEW.M.C. & POPPER, H. Sem. Liver Dis. 2, 136-146 (1984).
- 23 .: BEASLEY, R.P., HWANG L.Y. Sem. Liver Dis. 4, 113-121 (1984)
- 24 .: SAGUMA, K. et al. Hepatology 8, 1642-1646(1984).
- 25 .: HSU, T. et al. Cell 55, 627-635 (1988).
- 26 .: TOKINO, T. et al. J. Virol. 61, 3848-3854 (1987).
- 27 .: HINO, O., OHTAKE, K. & ROGLER, C.E.J. Virol. 63, 2638-2643 (1989).
- 28 .: ROGLER, C.E. et al. Science 230, 319-322 (1985).
- 29 .: PASQUINELLI, C. et al. J. Virol. 62. 629-632 (1988).
- 30 .: BLANQUET, V., GARREAU, F., CHENIVESSE, X., BRECHOT, C. & TURLEAU, C. Human Genetics 80, 274-276 (1988)
- 31 .: DEJEAN, A., BOUGUELERET, L., GRZESCHIK, K.H.&TIOLLAIS, P. Nature 322, 70-72 (1986).
- 32 .: DE THE, H.,MARCHIO, A., TIOLLAIS, P. & DEJEAN, A. Nature 330, 667-670 (1987).
- 33 .: MORGAN, D., KAPLAN, J.M., BISHOP, J.M. & VARMUS, H.E. Cell 57, 775-786 (1989).
- 34 .: SHENOY, S. et al. Cell 57, 763-774 (1989).
- 35 .: McVEY, D. et al. Nature 341,503-507 (1989).
- 36 .: BUCHKOVICH,K., DUFFY,L.A. & HARLOW, Ed. Cell 58, 1097-1105 (1989).
- 37 .: CHEN,P.L., SCULLY,P., SHEW,J.Y.L.&LEE,WH. Cell 58. 1193-1198 (1989).
- 38 .: DECAPRIO,J.A. et al. Cell 58, 1085-1095(1989).
- 39 .: SANGER,F.,NICKLEN, S. & COULSON, A.R. Proc. Nat. Acad. U.S.A. 74, 5463-5467 (1977).

Liste des séquences.

SEQ ID No. : 1
TYPE DE SEQUENCE : Nucléotide et sa protéine correspondante
LONGUEUR DE LA SEQUENCE : 1647 pb
NOMBRE DE BRINS : simple
CONFIGURATION : linéaire
TYPE DE MOLECULE : ADNc
ORIGINE
ORGANISME : humain
CARACTERISTIQUES : 1293 paires de bases peptide.
PROPRIETES : codant pour la cycline A humaine.

1 CGGCAGCCAGCCTATTCTTTGGCCGGGTCGGTGCGAGTGGTCGGCTGGGCAGAGTGCAC
60 GCTGCTTGGCGCCGCACGTGATCCCGCCGTCCACTCCCGGGAGCAGTGATGTTGGGCAAC
M L G N 4

120 TCTGCGCCGGGGCCTGCGACCCGCGAGGCGCTCGGCGCTGCTAGCATTGCAGCAGACGGC
S A P G P A T R E A L G A A S I A A D G 24

180 GCTCCAAGAGGACCAGGAGAATATCAACCCGGAAAAGGCAGCGCCCGTCCAACAACCGCG
A P R G P G E Y Q P G K G S A R P T T A 44

240 GACCCGGGCGCGCTGGCGGTACTGAAGTCCGGGAACCCGCGGGGTCTAGCGCACGAGCAG
D P G A L A V L K S G N P R G L A H E Q 64

300 AGGCCGAAGACGAGACGGGTTGCACCCCTTAAGGATCTTCCTGTAAATGATGAGCATGTC
R P K T R R V A P L K D L P V N D E H V 84

360 ACCGTTCCTCCTTGGAAAGCAAACAGTAAACAGCCTGCGTTCACCATTCATGTGGATGAA
T V P P W K A N S K Q P A F T I H V D E 104

420 GCAGAAAAAGAAGCTCAGAAGAAGCCAGCTGAATCTCAAAAAATAGAGCGTGAAGATGCC
A E K E A Q K K P A E S Q K I E R E D A 124

480 CTGGCTTTTAATTCAGCCATTAGTTTACCTGGACCCAGAAAACCATTGGTCCCTCTTGAT
L A F N S A I S L P G P R K P L V P L D 144

540 TATCCAATGGATGGTAGTTTTGAGTCACCACATACTATGGACATGTCAATTGTATTAGAA
Y P M D G S F E S P H T M D M S I V L E 164

600 GATGAAAAGCCAGTGAGTGTTAATGAAGTACCAGACTACCATGAGGATATTCACACATAC
D E K P V S V N E V P D Y H E D I H T Y 184

660 CTTAGGGAAATGGAGGTTAAATGTAAACCTAAAGTGGGTTACATGAAGAAACAGCCAGAC
L R E M E V K C K P K V G Y M K K Q P D 204

720 ATCACTAACAGTATGAGAGCTATCCTCGTGGACTGGTTAGTTGAAGTAGGAGAAGAATAT
I T N S M R A I L V D W L V E V G E E Y 224

780 AAACTACAGAATGAGACCCTGCATTTGGCTGTGAACTACATTGATAGGTTCCTGTCTTCC
K L Q N E T L H L A V N Y I D R F L S S 244

840 ATGTCAGTGCTGAGAGGAAAACTTCAGCTTGTGGGCACTGCTGCTATGCTGTTAGCCTCA
M S V L R G K L Q L V G T A A M L L A S 264

900 AAGTTTGAAGAAATATACCCCCCAGAAGTAGCAGAGTTTGTGTACATTACAGATGATACC
K F E E I Y P P E V A E F V Y I T D D T 284

960 TACACCAAGAAACAAGTTCTGAGAATGGAGCATCTAGTTTTGAAAGTCCTTACTTTTGAC
Y T K K Q V L R M E H L V L K V L T F D 304

1020 TTAGCTGCTCCAACAGTAAATCAGTTTCTTACCCAATACTTTCTGCATCAGCAGCCTGCA
L A A P T V N Q F L T Q Y F L H Q Q P A 324

1080 AACTGCAAAGTTGAAAGTTTAGCAATGTTTTTGGGAGAATTAAGTTTGATAGATGCTGAC
N C K V E S L A M F L G E L S L I D A D 344

1140 CCATACCTCAAGTATTTGCCATCAGTTATTGCTGGAGCTGCCTTTCATTTAGCACTCTAC
P Y L K Y L P S V I A G A A F H L A L Y 364

1200 ACAGTCACGGGACAAAGCTGGCCTGAATCATTAATACGAAAGACTGGATATACCCTGGAA
T V T G Q S W P E S L I R K T G Y T L E 384

1260 AGTCTTAAGCCTTGTCTCATGGACCTTCACCAGACCTACCTCAAAGCACCACAGCATGCA
S L K P C L M D L H Q T Y L K A P Q H A 404

1320 CAACAGTCAATAAGAGAAAAGTACAAAAATTCAAAGTATCATGGTGTTTCTCTCCTCAAC
Q Q S I R E K Y K N S K Y H G V S L L N 424

1380 CCACCAGAGACACTAAATCTGTAACAATGAAAGACTGCCTTTGTTTTCTAAGATGTAAAT
P P E T L N L * (431)

1440 CACTCAAAGAGTATGGTGTACAGTTTTTAACTTAGGTTTTTAATTTTACAATCATTTCTG
1500 AATACAGAAGTTGTGGCCAAGTACAAATTATGGTATCTATTACTTTTTAAATGGTTTTAA
1560 TTTGTATATCTTTTGTATATGTATCTGTCTTAGATATTTGGCTAATTTTAAGTGGTTTTG
1620 TTAAAGTATTAATGATGCCAGCTGCCG

## Revendications

1. Procédé de diagnostic *in vitro* de la prolifération cellulaire, **caractérisé en ce que**, à partir d'un échantillon humain de cellules ou de tissus, on détermine le taux de cycline A humaine.

2. Procédé selon la revendication 1, **caractérisé en ce que** l'on détecte la cycline A à l'aide d'anticorps ou d'anti-sérum anti-cycline A.

3. Procédé selon la revendication 1, **caractérisé en ce que** l'on détecte la cycline A à l'aide d'anticorps polyclonaux ou monoclonaux anti-cycline A humaine.

4. Procédé selon la revendication 1, 2 ou 3, **caractérisé en ce qu'**il comprend en plus une opération de quantification de la cycline A présente dans un échantillon étudié.

5. Procédé selon la revendication 4, **caractérisé en ce que** la quantification de la cycline A est effectuée par Western Blot, immunohistochimie ou comptage des noyaux cellulaires.

6. Procédé selon l'une quelconque des revendications 1 à 5, appliqué à la détection ou au diagnostic de la prolifération cellulaire.

7. Préparation d'anticorps anti-cycline A humaine, capables de détecter la cycline A humaine pour la mise en oeuvre du procédé selon l'une quelconque des revendications 1 à 6.

8. Préparation selon la revendication 7, **caractérisée en ce que** les anticorps sont des anticorps polyclonaux.

9. Préparation selon la revendication 7, **caractérisée en ce que** les anticorps sont des anticorps monoclonaux.

10. Anti-sérum anti-cycline A humaine, capable de détecter la cycline A humaine, pour la mise en oeuvre du procédé selon l'une quelconque des revendications 1 à 6.

11. ADNc anti-sens complémentaire de la séquence de la cycline A humain, capable d'inhiber la prolifération cellulaire.

12. Plasmide incluant l'ADNc anti-sens selon la revendication 11.

13. Vecteur rétroviral incluant l'ADNc anti-sens selon la revendication 11.

14. Liposome comprenant l'ADNC anti-sens selon la revendication 11.

## Patentansprüche

1. In-vitro-Verfahren zur Diagnostik der Zellproliferation, dadurch charakterisiert, dass man ausgehend von einer Probe von humanen Zellen oder Geweben die Menge an Human-Cyclin A bestimmt,

2. , Verfahren nach Anspruch 1, dadurch charakterisiert, dass man Cyclin A mit Hilfe von Anti-Cyclin A-Antikörpern oder -Antiserum bestimmt,

3. Verfahren nach Anspruch 1, dadurch charakterisiert, dass man das Cyclin A mit Hilfe von polyklonalen oder monoklonalen humanen Anti-Cyclin A-Antikörpern bestimmt.

4. Verfahren nach Anspruch 1, 2 oder 3, dadurch charakterisiert, dass es darüber hinaus eine Mengenbestimmung des in der untersuchten Probe vorhandenen Cyclin A umfasst.

5. Verfahren nach Anspruch 4, dadurch charakterislert, dass die Mengenbestimmung von Cyclin A durch Western Blot, Immunohistochemie oder Zählen von Zellkernen durchgeführt wird.

6. Verfahren nach einem der Ansprüche 1 bis 5, das zum Nachwels oder zur Diagnostik der Zellproliferation angewendet wird.

7. Präparat von Human-Antl-Cyclin A-Antikörpern, die zum Nachweis von Human-Cyclin A fähig sind, zur Durchführung des Verfahrens nach einem der Ansprüche 1 bis 6.

8. Präparat nach Anspruch 7, dadurch charakterisert, dass die Antlkörper polyklonale Antlkörper sind.

9. Präparat nach Anspruch 7, dadurch charakterisiert, dass die Antikörper monoklonale Antikörper sind,

10. Human-Anti-Cyclin A-Antiserum, das zum Nachweis von Human-Cyclin A fähig ist, zur Durchführung des Verfahrens nach einem der Ansprüche 1 bis 6.

11. Zur Sequenz von Human-Cyclin A komplementäre Antlsense-cDNA, die zur Inhibierung der Zellproliferation fähig ist.

12. Plasmid, das die Antisense-cDNA nach Anspruch 11 enthält.

13. Retroviraler Vektor, der die Antisense-cDNA nach Anspruch 11 enthält.

14. Liposom, das die Antisense-cDNA nach Anspruch 11 umfaßt,

## Claims

1. Method for the *in vitro* diagnosis of cell proliferation, **characterized in that**, starting with a human cell or tissue sample, the level of human cyclin A is determined.

2. Method according to Claim 1, **characterized in that** cyclin A is detected using anti-cyclin A antibody or antiserum.

3. Method according to Claim 1, **characterized in that** oyclin A is detected using anti-human-cyclin A polyclonal or monoclonal antibodies.

4. Method according to Claim 1, 2 or 3, **characterized in that** it comprises, in addition, an operation for quantifying the cyclin A present in a sample under study.

5. Method according to Claim 4, **characterized in that**. the quantification of cyclin A is carried out by Western blotting, immunohistochemistry or counting of cell nuclei.

6. Method according to any one of Claims 1 to 5, applied to the detection or diagnosis of cell proliferation.

7. Preparation of anti-human-cyclin A antibodies capable of detecting human cyclin A for carrying out the method according to any one of Claims 1 to 6.

8. Preparation according to Claim 7, **characterized in that** the antibodies are polyclonal antibodies.

9. Preparation according to Claim 7, **characterized in that** the antibodies are monoclonal antibodies.

10. Anti-human-cyclin A antiserum, capable of detecting human cyclin A, for carrying out the method according to any one of Claims 1 to 6.

11. Anti-sense cDNA complementary to the human cyclin A sequence capable of inhibiting cell proliferation.

12. Plasmid including the anti-sense cDNA according to Claim 11.

13. Retroviral vector including the anti-sense cDNA according to Claim 11.

14. Liposome comprising the anti-sense cDNA according to Claim 11.
